(19) Europäisches Patentamt
European Patent Office
Office européen des brevets

(11) Numéro de publication : 0 255 904 B1

(12) # FASCICULE DE BREVET EUROPEEN

(45) Date de publication du fascicule du brevet : 02.01.91 Bulletin 91/01

(51) Int. Cl.[5] : C07C 47/32, C07C 45/54

(21) Numéro de dépôt : 87110800.7

(22) Date de dépôt : 25.07.87

(54) **Procédé pour la préparation de dihydrocyclocitral.**

(30) Priorité : 06.08.86 CH 3154/86

(43) Date de publication de la demande : 17.02.88 Bulletin 88/07

(45) Mention de la délivrance du brevet : 02.01.91 Bulletin 91/01

(84) Etats contractants désignés : CH DE FR GB LI NL

(56) Documents cités : FR-A- 1 204 407

(73) Titulaire : FIRMENICH SA
1, route des Jeunes Case Postale 239
CH-1211 Genève 8 (CH)

(72) Inventeur : Simmons, Dana Philip, Dr.
7, rue Guye
CH-1203 Geneve (CH)

(74) Mandataire : Salvadori, Giuseppe, Dr.
c/o Firmenich S.A. Case Postale 239
CH-1211 Genève 8 (CH)

EP 0 255 904 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

**Description**

Le 2,2,6-triméthyl-cyclohexanecarboxaldéhyde, également connu sous le nom de dihydrocyclocitral, est un aldéhyde cycloaliphatique de formule

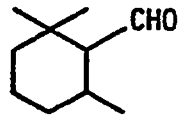

Il représente un composé intermédiaire utile dans de nombreuses synthèses organiques et sert en tant que matière première à la préparation de différents produits intéressant l'industrie des arômes et de la parfumerie, ainsi qu'à la préparation de produits pharmaceutiques de la série des caroténoïdes et stéroïdes entre autres (voir à cet effet : demande de brevet européen n° 118'809, publiée le 19.9.84).

L'art antérieur fait état de plusieurs méthodes pour sa préparation.

M. de Botton [Bull. Soc. Chim. de France, 33, 2466-73 (1966)] a décrit la préparation du dihydrocyclocitral à partir de 2,2,6-triméthyl-cyclohexanone suivant le schéma réactionnel que voici :

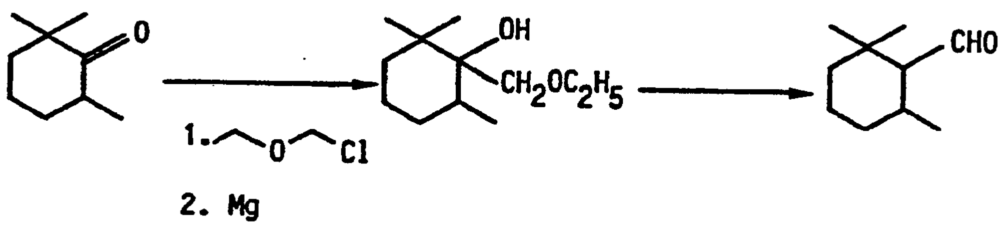


D'autres méthodes plus anciennes font appel à l'hydrogénation du cyclocitral [Helv. Chim. Acta, 31, 417 (1948) et Helv. Chim. Acta, 34, 1160 (1951)] ou à l'oxydation du dihydrocyclogéraniol [Helv. Chim. Acta, 23, 529 (1940) et Helv. Chim. Acta, 23, 1265 (1940)]. Le remplacement d'un atome d'oxygène cétonique par un hydrogène et un groupe formyle du type

$$R_2CO \longrightarrow R_2CHCHO$$

constitue une approche importante en synthèse organique pour laquelle plusieurs types de réactifs ont été développés. Toutefois, parmi ceux-ci, aucun ne donne des résultats satisfaisants pour la transformation de cétones stériquement encombrées ayant un atome d'hydrogène en position alpha du groupe cétonique. E. J. Corey et M. A. Tius [Tetrahedron Letters, 21, 3535-8 (1980)] ont décrit une méthode de préparation du dihydrocyclocitral à partir de 2,2,6-triméthyl-cyclohexanone à l'aide précisément d'un de ces réactifs particuliers, la diphényl-méthoxy-méthyl phosphine. La nature du réactif rend cependant difficile l'application de cette méthode à une préparation industrielle.

Nous avons maintenant découvert qu'il était possible d'obtenir le dihydrocyclocitral à l'aide d'un procédé simple et économique, lequel procédé fait appel à des réactifs traditionnels. La présente invention a trait à un procédé pour la préparation du dihydrocyclocitral qui consiste en la cyclisation au moyen d'un agent de cyclisation acide d'un énol ester de formule

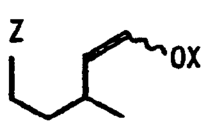

(I)

dans laquelle la ligne ondulée définit une liaison C-O de configuration cis ou trans, X représente un groupe acyle ou P(O) $(OR)_2$, R étant un radical monovalent alkyle inférieur ou aryle, et Z définit un groupe monovalent de formule

a. $CH=C(CH_3)_2$,
b. $CH_2-C(OH)(CH_3)_2$, ou
c. $CH_2-C(CH_3)=CH_2$.

Ainsi le radical R peut représenter un groupe alkyle $C_1$-$C_6$, par exemple méthyle, éthyle, propyle ou isopropyle ou un groupe phényle et X représente un groupe acyle du type R'CO, R' étant de préférence un radical alkyle inférieur de $C_1$ à $C_4$.

Suivant un mode d'exécution préférentiel, l'on effectue la cyclisation sur l'acétate de 3,7-diméthyl-octa-1,6-diène-1-yle, l'acétate de 3,7-diméthyl-7-hydroxy-oct-1-ène-1-yle ou l'acétate de 3,7-diméthyl-octa-1,7-diène-1-yle. Ces esters, tout comme les autres esters dialkylphosphates définis par la formule (I), peuvent être préparés à partir de citronellal ou d'hydroxycitronellal selon un procédé analogue à ceux décrits dans la littérature [voir par exemple : J. Am. Chem. Soc., 72, 2617 (1950)]. Par exemple, on peut effectuer cette préparation à l'aide d'un traitement du citronellal, respectivement hydroxycitronellal avec l'anhydride acétique en présence d'un agent basique, telle une amine tertiaire comme la triéthylamine ou en présence d'un carbonate alcalin, par exemple le carbonate de potassium.

L'acétate de 3,7-diméthyl-octa-1,7-diène-1-yle peut être préparé par déshydratation de l'hydroxycitronellal, suivie d'estérification, selon des techniques en soi connues.

A titre d'agents de cyclisation acides, on peut utiliser les acides protoniques organiques ou minéraux ou les acides du type de Lewis. Parmi les agents préférentiels, il convient de citer les acides sulfurique, phosphorique, polyphosphorique, méthanesulfonique, acétique, trifluoroacétique ou encore, parmi les acides de Lewis, le tétrachlorure d'étain, le tétrachlorure de titanium ou le trifluorure de bore par exemple.

La température à laquelle on effectue la réaction de cyclisation n'est pas critique et peut varier dans une gamme de valeurs assez étendue. Elle est choisie généralement en fonction de l'agent acide utilisé. C'est ainsi que des bons rendements en produit final ont été obtenus à 0°C en utilisant, comme agent acide, l'acide sulfurique et, comme ester de départ, le diéthylphosphate de 3,7-diméthyl-octa-1,6-diène-1-yle. De très bons rendements ont également été obtenus par traitement à 100°C de l'acétate de 3,7-diméthyl-octa-1,6-diène-1-yle avec l'acide phosphorique concentré ou l'acide polyphosphorique. Des températures supérieures ou inférieures aux valeurs indiquées peuvent également être employées.

Des modes particuliers d'exécution seront indiqués dans les exemples spécifiques de préparation donnés ci-après.

Comme il a été indiqué plus haut, le procédé de l'invention présente des avantages certains vis-à-vis des procédés connus de l'art antérieur quant à la simplicité des opérations requises et à leur économie. A cela s'ajoute un avantage majeur : grâce au procédé de la présente invention, il est en effet désormais possible de préparer du dihydrocyclocitral optiquement actif dans ses deux formes épimères de formule

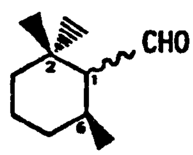

et

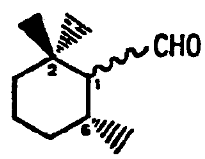

(a) (b)

Or, ces deux antipodes constituent des produits nouveaux dont la préparation n'était point possible à l'aide des procédés connus à ce jour.

L'invention a donc également pour objet lesdits antipodes optiquement actifs. Ces derniers représentent des produits de départ particulièrement utiles pour l'obtention de composés à leur tour optiquement actifs et dont l'intérêt réside dans le fait que leurs propriétés diffèrent, dans une mesure plus ou moins prononcée, de celle des composés racémiques correspondants.

Tel est le cas pour bon nombre de composés biologiquement actifs pour lesquels les centres récepteurs physiologiques de l'homme ou des animaux sont à même de différencier la nature propre de chaque énantiomère, par exemple les centres de la perception olfactive.

En l'état actuel de nos connaissances, il n'y a pas de théorie univoque apte à élucider le phénomène de la perception olfactive. Toutefois, il est souvent apparu à l'expérience que seul l'une des formes optiquement actives était dotée des propriétés odorantes souhaitées, le racemate montrant au plus la moitié de l'intensité sans toutefois posséder le même caractère olfactif. D'autre part, les méthodes de séparation classiques de la forme racémique en ses composants D ou L conduisent au plus à un rendement de 50%, ce qui se traduit par un rapport économique défavorable. La préparation d'antipodes à l'état pur revêt par conséquent une importance majeure.

Les épimères (a) et (b) sont obtenus par le procédé de l'invention à partir des énol esters de formule (I) optiquement actifs. En effet, la cyclisation selon l'invention s'accompagne de façon inattendue de la

rétention de la configuration de l'atome de carbone asymétrique en position 6. A partir de (+)- ou (–)-citronellal, l'on obtiendra donc l'antipode correspondant du dihydrocyclocitral.

Le (+)- et le (–)-citronellal sont des constituants actifs dans plusieurs huiles essentielles à partir desquelles ils peuvent être extraits, les deux produits sont par ailleurs disponibles sur le marché à des degrés de pureté variables. Le R(+)-citronellal peut par exemple être obtenu de la citronelle de Chine, accompagné de quantités mineures de linalol et d'isopulegol.

L'invention est illustrée à l'aide des exemples suivants dans lesquels les températures sont indiquées en degrés centigrades et les abréviations ont le sens usuel dans l'art.

Exemple 1

(±)-Dihydrocyclocitral à partir de (±)-citronellal

a. 167 Kg d'anhydride acétique et 77 kg de carbonate de potassium ont été portés à 80° dans un réacteur équipé d'une pompe d'introduction et d'un réfrigérant de condensation. 85 kg de (±)-citronellal ont été ensuite ajoutés au moyen de la pompe doseuse d'introduction pendant 40 min. et le mélange a été porté à reflux pendant 7 h. Après refroidissement, 100 kg de toluène et 500 l d'eau ont été ajoutés au mélange de réaction et, après séparation, la phase organique a été lavée à l'eau (100 l) pour fournir une solution dans le toluène de l'acétate de 3,7-diméthyl-octa-1,6-diène-1-yle accompagné d'une quantité mineure du diester de formule

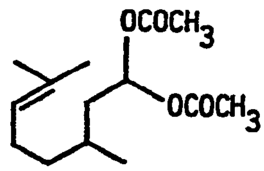

(II)

b. A la solution ainsi obtenue, on a ensuite ajouté 100 kg de toluène et 200 kg d'acide phosphorique à 85% et le mélange résultant a été chauffé sous agitation à 100° pendant 1 3/4 h, période au cours de laquelle le produit de départ a été complètement transformé. Le mélange de réaction a été refroidi à 15° et 347 l d'eau y ont été ajoutés. Après décantation, la phase organique a été lavée avec 100 l de solution aqueuse de $NaHC0_3$ à 6% et le toluène a été éloigné par distillation (temp. 60-80°/150-110 mb). 91 Kg de dihydrocyclocitral brut ont pu ainsi être obtenus. Une purification ultérieure par distillation sur résidu (57-73°/30-9 mb) a permis l'obtention de 75,4 kg de (±)-dihydrocyclocitral dont la pureté était de 68,1% (rend. 60,4% en produit pur).

Exemple 2

(±)-Dihydrocyclocitral à partir de (±)-citronellal

a. Un mélange constitué par 112,6 kg d'anhydride acétique, 8,5 kg d'acétate de potassium et 56 kg de triéthylamine a été chauffé à 80° et à cette température on y a ajouté 84,5 kg de (±)-citronellal (temps d'addition : 40 min). Le tout a été maintenu à reflux pendant 7 h. Après refroidissement à température ambiante, on y a ajouté 100 kg de toluène et 350 l d'eau et les deux phases ont été séparées. L'énol acétate désiré, accompagné d'une quantité mineure du diacétate de formule (II) a été ainsi obtenu sous forme de solution dans le toluène.

b. A cette solution, diluée par l'addition de 100 kg de toluène, on a ajouté sous agitation 200 kg d'acide phosphorique à 85%, puis le mélange a été chauffé à 100° pendant 1 h 40 min. Après refroidissement à 15°, 300 l d'eau y ont été ajoutés et la phase organique a été lavée avec une solution aqueuse de $NaHC0_3$ à 6%. Le toluène a été éloigné par distillation sous pression réduite (45-88°/200-100 mb) pour fournir 92,2 kg de (±)-dihydrocyclocitral brut. Une distillation sur résidu (40-60°/40-50 mb) a permis l'obtention de 76,6 kg dont la pureté était de 65,7% (rend. 65,6% en produit pur).

Le tableau suivant résume les résultats observés lors des essais (Exemples 3 à 10) effectués au moyen d'autres réactifs acides de cyclisation.

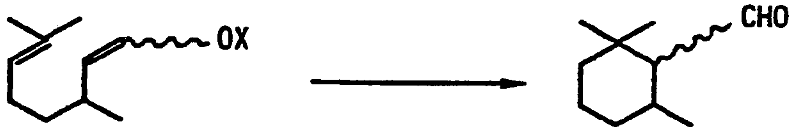

TABLEAU

| Exemple | X | Acide | Temp. [°C] | Rend. théor. [%] |
|---|---|---|---|---|
| 3 | $-P(O)(OC_2H_5)_2$ | $H_2SO_4$ | 0° | 60 |
| 4 | $CH_3CO$ | $H_2SO_4$ | 0° | 35 |
| 5 | $CH_3CO$ | $CH_3SO_3H$ | 0° | 42 |
| 6 | $CH_3CO$ | $TiCl_4$ | 0° | 33 |
| 7 | $CH_3CO$ | $BF_3.O(C_2H_5)_2$ | 0° | 55 |
| 8 | $CH_3CO$ | $H_3PO_4$ 85% | 100° | 74 |
| 9 | $CH_3CO$ | PPA | 100° | 72 |
| 10 | $CH_3CO$ | $F_3CCO_2H$ | 80° | 40 |

Exemple 11

(+)-Dihydrocyclocitral

Un mélange constitué par 100 g de (+)-citronellal, ayant un alpha$^{20}_D$ = +9,4°, 132,5 g d'anhydride acétique, 65,6 g de triéthylamine et 10 g d'acétate de potassium a été chauffé à 120° pendant 6 h. Après avoir été refroidi à température ambiante, on y a ajouté 230 g de toluène, puis il a été lavé une fois à l'eau et une fois avec une solution aqueuse de $NaHCO_3$. La phase organique a été ensuite versée sur une solution aqueuse (230 g) d'acide phosphorique à 85% et le tout a été chauffé à 100° pendant 90 minutes.

Après refroidissement, le mélange a été lavé avec de l'eau, une solution aqueuse de bicarbonate de sodium et concentré. Une simple distillation a permis d'obtenir 73,5 g d'un produit ayant une teneur de 87% de dihydrocyclocitral (76% trans, 11% cis), alpha$^{20}_D$ = +0,04° (pur). Une purification ultérieure au moyen de distillation sur une colonne de type Fischer a permis d'obtenir un produit pur dont le contenu en isomère trans était de 93% ; alpha$^{20}_D$ = +0,05° (pur).

Exemple 12

(–)-Dihydrocyclocitral

Le S(–)-citronellal, obtenu par oxydation de S(–)-citronellol (origine : Fluka AG, Buchs, Suisse) a été transformé en (–)-dihydrocyclocitral suivant un procédé identique à celui décrit dans l'exemple 11 ci-dessus. Le produit obtenu a montré un alpha$^{20}_D$ = –0,06° (pur).

Exemple 13

(±)-Dihydrocyclocitral à partir de 3,7-diméthyl-oct-7-énal

a. Un mélange constitué par 16 g (0,16 M) d'anhydride acétique, 1,2 g d'acétate de potassium et 7,8

g (0,078 M) de triéthylamine, auquel on a ajouté 12 g (0,078 M) de 3,7-diméthyl-oct-7-énal, a été maintenu à reflux pendant 6 heures. Après refroidissement à température ambiante, on a ajouté de l'eau et du toluène (40 ml) et les deux phases ont été séparées.

b. La phase organique a été mélangée avec 30 g d'acide phosphorique à 85% et le tout a été chauffé à reflux pendant deux heures. Après refroidissement à température ambiante, on a ajouté de l'eau et la phase organique a été séparée, concentrée puis distillée dans un appareil à boules (temp. du bain 150°/15-20 mb). On a ainsi obtenu 9,5 g de (±)-dihydrocyclocitral dont la pureté était de 74%.

Le 3,7-diméthyl-oct-7-énal, utilisé comme produit de départ dans le procédé décrit ci-dessus, a été préparé par déshydratation au moyen de $KHSO_4$ d'hydroxycitronellal.

Exemple 14

(±)-Dihydrocyclocitral à partir d'hydroxycitronellal

En opérant comme indiqué à l'Exemple 13, on a préparé le produit désiré en utilisant les produits et réactifs suivants :

| | | |
|---|---|---|
| a. | hydroxycitronellal | 25 g |
| | triéthylamine | 37 g |
| | anhydride acétique | 75 g |
| | acétate de potassium | 2,5 g |
| b. | $H_3PO_4$ à 85% | 60 g |

22,3 G de (±)-dihydrocyclocitral ont été ainsi obtenus (pureté : 63%).

## Revendications

1. Procédé pour la préparation de dihydrocyclocitral caractérisé en ce qu'on cyclise au moyen d'un agent de cyclisation acide un énol ester de formule

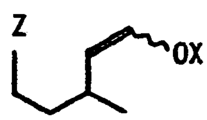

(I)

dans laquelle la ligne ondulée définit une liaison C-O de configuration cis ou trans, X représente un groupe acyle ou P(O) $(OR)_2$, R étant un radical monovalent alkyle inférieur ou aryle, et Z définit un groupe monovalent de formule

a. $CH=C(CH_3)_2$,

b. $CH_2-C(OH)(CH_3)_2$, ou

c. $CH_2-C(CH_3)=CH_2$.

2. Procédé selon la revendication 1 caractérisé en ce qu'on utilise comme produit de départ l'acétate de 3,7-diméthyl-octa-1,6-diène-1-yle, l'acétate de 3,7-diméthyl-octa-1,7-diène-1-yle ou l'acétate de 3,7diméthyl-7-hydroxy-oct-1-ène-1-yle.

3. Procédé selon la revendication 1 caractérisé en ce qu'on utilise comme produit de départ le phosphate diéthylique de 3,7-diméthyl-octa-1,6-diène-1-yle, le phosphate diéthylique de 3,7-diméthyl-octa-1,7-diène-1-yle ou le phosphate diéthylique de 3,7-diméthyl-7-hydroxy-oct-1-ène-1-yle.

4. Procédé selon la revendication 1 caractérisé en ce qu'on utilise à titre d'agent de cyclisation acide un acide protonique organique ou minéral ou un acide du type de Lewis.

5. Procédé selon la revendication 4 caractérisé en ce qu'on utilise à titre d'agent de cyclisation l'acide acétique, l'acide trifluoroacétique, l'acide phosphorique, l'acide méthanesulfonique, le trifluorure de bore ou le tétrachlorure de titanium.

6. Procédé selon la revendication 5 caractérisé en ce qu'on effectue la cyclisation à une température

comprise entre 0° et 100°C.

7. Dihydrocyclocitral sous forme d'un des antipodes optiquement actifs de formules

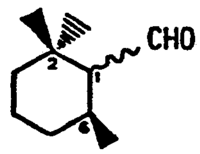

et

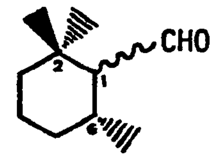

(a) (b)

8. Procédé selon la revendication 1 caractérisé en ce qu'on utilise à titre d'énol ester de formule (I) l'un des composés optiquement actifs de formules

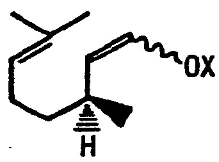

ou

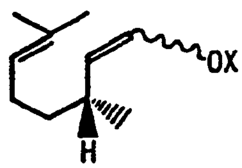

(Ia) (Ib)

dans lesquelles X et la ligne ondulée ont le sens indiqué à la revendication 1 et que l'on obtient le dihydrocyclocitral sous forme de l'un des antipodes définis à la revendication 7.

**Ansprüche**

1. Verfahren zur Herstellung von Dihydrocyclocitral, dadurch gekennzeichnet, daß man mittels eines sauren Cyclisierungsmittels einen Enolester der Formel,

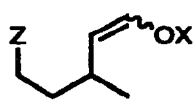

(I)

in welcher die wellige Linie eine cis oder trans C-O Einfachbindung darstellt, X eine Acylgruppe oder P(O)$(OR)_2$, worin R für einen einwertigen niedrigen Alkylrest oder einen Arylrest steht, bedeutet und Z eine Gruppe der Formel

a. $CH=C(CH_3)_2$,

b. $CH_2-C(OH)(CH_3)_2$, oder

c. $CH_2-C(CH_3)=CH_2$ bezeichnet, cyclisiert.

2. Verfahren gemäß Anspruch 1, dadurch gekennzeichnet, daß man als Ausgangprodukt 3,7-Dimethyl-octa-1,6-dien-1-yl Acetat, 3,7-Dimethyl-octa-1,7-dien-1-yl Acetat oder 3,7-Dimethyl-7-hydroxy-oct-1-en-1-yl Acetat verwendet.

3. Verfahren gemäß Anspruch 1, dadurch gekennzeichnet, daß man als Ausgangprodukt 3,7-Dimethyl-octa-1,6-dien-1-yl Diäthylphosphat, 3,7-Dimethyl-octa-1,7-dien-1-yl Diäthylphosphat oder 3,7-Dimethyl-7-hydroxy-oct-1-en-1-yl Diäthylphosphat verwendet.

4. Verfahren gemäß Anspruch 1, dadurch gekennzeichnet, daß man als saures Cyclisierungsmittel eine protonische organische oder mineralische Säure oder eine Lewissäure verwendet.

5. Verfahren gemäß Anspruch 4, dadurch gekennzeichnet, daß man als saures Cyclisierungsmittel eine Essigsäure, Trifluoroessigsäure, Phosphorsäure, Methansulfonsäure, Bortrifluorid oder Titantetrachlorid verwendet.

6. Verfahren gemäß Anspruch 5, dadurch gekennzeichnet, daß die Cyclisierungsreaktion bei einer Temperatur von 0° bis 100°C durchgeführt wird.

7. Dihydrocyclocitral als einer der optischen Antipoden der Formeln

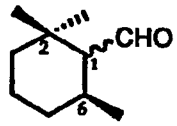

und

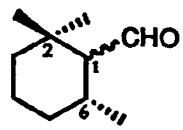

(a) (b)

8. Verfahren gemäß Anspruch 1, dadurch gekennzeichnet, daß man als Enolester der Formel (I) eine der optisch aktiven Verbindungen der Formeln,

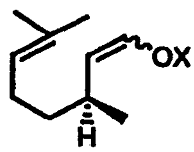

oder

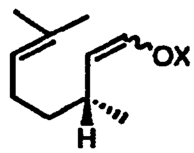

(Ia) (Ib)

in welchen X und die wellige Linie die im Anspruch 1 angegebener Bedeutung haben, verwendet, und, daß man Dihydrocyclocitral als einen der im Anspruch 7 angegebenen Antipoden erhält.

## Claims

1. Process for the preparation of dihydrocyclocitral, characterized by the cyclization, by means of an acidic cyclization agent, of an enol of formula

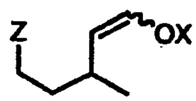

(I)

wherein the wavy line stands for a C-O bond of cis or trans configuration, X represents an acyl radical or P(O) $(OR)_2$, R being a lower alkyl monovalent radical or an aryl, and Z represents a monovalent radical of formula

a. $CH = C(CH_3)_2$,
b. $CH_2- C(OH)(CH_3)_2$, or
c. $CH_2- C(CH_3)=CH_2$.

2. Process according to claim 1, characterized in that 3,7-dimethyl-octa-1,6-dien-1-yl acetate, 3,7-dimethyl-octa-1,7-dien-1-yl acetate or 3,7-dimethyl-7-hydroxy-oct-1-ene-yl acetate is used as starting product.

3. Process according to claim 1, characterized in that 3,7-dimethyl-octa-1,6-dien-1-yl diethylphosphate, 3,7-dimethyl-octa-1,7-dien-1-yl diethylphosphate or 3,7-dimethyl-7-hydroxy-oct-1-en-1-yl diethylphosphate is used as starting product.

4. Process according to claim 1, characterized in that the acidic cyclization agent is a mineral or organic protonic acid or a Lewis type acid.

5. Process according to claim 4, characterized in that the acidic cyclization agent is acetic acid, trifluoroacetic acid, phosphoric acid, methane-sUlfonic acid, boron trifluoride or titanium tetrachloride.

6. Process according to claim 5, characterized in that the cyclization is carried out at a temperature of between 0° and 100°C.

7. Dihydrocyclocitral in the form of one of the optically active antipodes of formulae

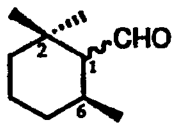

and

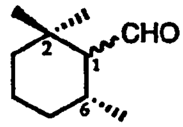

(a) (b)

8. Process according to claim 1, characterized in that the enol ester of formula (I) is one of the optically active compounds of formulae

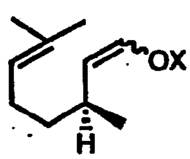

or

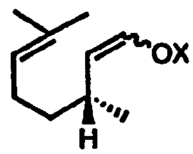

(Ia) (Ib)

wherein X and the wavy line have the meaning set forth in claim 1, and in that the obtained dihydrocyclocitral occurs in the form of one of the antipodes defined in claim 7.